# EUROPEAN PATENT SPECIFICATION

(11) **EP 3 283 066 B1**
(45) Date of publication and mention of the grant of the patent: **11.05.2022**
(21) Application number: 15716808.9
(22) Date of filing: 16.04.2015
(51) Int. Cl.: A61K 31/216, A61K 31/223, A61P 1/16, A61P 1/00, A61P 9/14, A61P 9/10, A61P 15/02, A61P 17/06, A61P 27/16, A61P 25/24, A61K 9/00, A61P 1/04, A61P 9/00, A61P 17/02, A61P 25/00, A61P 29/00, A61K 31/60

(54) **4-PHENYLBUTYRIC ACID DERIVATIVES**
4-PHENYLBUTTERSÄUREDERIVATE
DÉRIVÉS D'ACIDE 4-PHÉNYLBUTYRIQUE

(43) Date of publication of application: 21.02.2018
(73) Proprietor: Phenotec AG, 6302 Zug (CH)
(72) Inventor: Truog-Fromer, Christine, 7000 Chur (CH)
(74) Representative: Hepp Wenger Ryffel AG
(86) International application number: PCT/EP2015/058329
(87) International publication number: WO 2016/165770

(56) References cited:
- EP-A1- 2 389 932
- EP-A1- 2 599 767
- T. A. GUDASHEVA ET AL: "Design and synthesis of cholecystokinin-4 dipeptide analogues with anxiolytic and anxiogenic activities", RUSSIAN JOURNAL OF BIOORGANIC CHEMISTRY, vol. 33, no. 4, 1 July 2007 (2007-07-01), pages 383-389, XP055235170, RU ISSN: 1068-1620, DOI: 10.1134/S1068162007040036

## Description

The present invention relates to 4-phenylbutyric acid derivatives according to the independent claims.

Inflammatory bowel diseases are a group inflammatory conditions of the colon and the small intestine. Crohn's disease and ulcerative colitis are the principle types of inflammatory bowel diseases. It is important to note, however, that Crohn's disease cannot only affect the small intestine but also other parts of the gastrointestinal tract. Ulcerative colitis, on the other hand, primarily affects the colon and the rectum. Despite extensive research, the causes underlying inflammatory bowel diseases are not fully understood. It is increasingly thought that alterations to internal bacteria can contribute to inflammatory bowel diseases, as they seem to arrive as a result of a complex interaction between environmental and genetic factors. Even with various treatments available, inflammatory bowel diseases can cause a significant limitation of the quality of the life of patients. In 2013, inflammatory bowel diseases resulted in 51'000 deaths worldwide.

A disease related to ulcerative colitis is primary sclerosing cholangitis, (PSC). PSC is a disease of the bile ducts that causes inflammation and subsequent obstruction of the bile ducts inside and outside of the liver. The inflammation impedes the flow of bile to the gut, which can ultimately lead to cirrhosis of the liver, liver failure and liver cancer. More than 80% of those with PSC also have ulcerative colitis. The definitive treatment is a liver transplant. A study of 2007 estimated that the average survival time from the diagnosis of PSC to be approximately 25 years, and the medium time until either death or liver transplantation to be approximately 10 years.

Psoriasis is a common, chronic, relapsing/remitting systemic disease characterized by skin lesions including red, scaly patches, papules, and plugs, which usually itch. The skin lesions seen in psoriasis may vary in severity from minor localized patches to complete body coverage. The causes of psoriasis are not fully understood. It is not purely a skin disorder and can have a negative impact on many organ systems. Psoriasis has been associated with an increased risk of certain cancers, cardio vascular disease, and other disorders such as Crohn's disease and ulcerative colitis. No cure is available for psoriasis, but various treatments can help to control the symptoms. The disease affects 2-4% of the general population. Psoriasis is known to have a negative impact on the quality of life of both the affected person and the individual family members. Depending on the severity and location of outbreaks, individuals may experience significant physical discomfort and some disabilities. Itching and pain can interfere with basic functions, such as self-care and sleep.

Clinically, vaginitis is an inflammation of the vagina. It can result in discharge, itching and pain, and is often associated with an irritation or infection of the vulva. It may be due to infection and/or medical irritation. Vaginal infections left untreated can lead to further complications, especially for pregnant women. For bacterial vaginitis, these include premature delivery, postpartum infections, clinically apparent and subclinical pelvic inflammation disease, as well as postsurgical complications. Increased vulnerability to HIV infection and possible infertility are other complications. Furthermore, there is a persistent discomfort to the patients.

Angiopathic diseases are diseases of the blood vessels, such as arteries, veins and capillaries.

Varicose veins are veins that have become enlarged and twisted. The term commonly refers to veins on the leg, although varicose veins can occur elsewhere. Veins have pairs of leaflet valves to prevent blood from flowing backwards. Leg muscles pump the veins to return blood to the heart, against the effect of gravity. When veins become varicose, the leaflets of the valves no longer meet properly, and the valves do not work. This allows blood to flow backwards and the veins enlarge even more. Varicose veins are most common in the superficial veins of the legs, which are subject to high pressure when standing. Besides being a cosmetic problem, varicose veins can be painful, especially when standing. Severe long-standing varicose veins can lead to leg swelling, venous eczema, skin thickening and also ulceration.

Major depressive disorder is a mental disorder characterized by a pervasive and persistent low mood that is accompanied by low self-esteem and by a loss of interest or pleasure in normally enjoyable activities. Major depressive disorder is a disabling condition that adversely affects a person's family, work or school-life, sleeping and eating habits, and general health. In the United States, around 3.4% of people with a major depression commit suicide, and up to 60% of people who commit suicide have depression or another mood disorder.

Tinnitus is the perception of sound within the human ear when no external sound is present. Tinnitus is a condition that can result from a wide range of underlying causes. The most common cause is noise-induced hearing loss. Other causes include neurological damage, ear infections, oxidative stress, emotional stress, foreign objects in the ear, nasal allergies that prevent fluid drain, earwax build-up, and exposure to loud sounds. Tinnitus may be an accompaniment of sensorineural hearing loss or congenital hearing loss, or may be observed as a side-effect of certain medications. Despite the fact that 10-15% of the population is affected by Tinnitus, there is no effective medication.

4-phenylbutyric acid is a well-known compound that is marketed in form of its sodium salt as a drug in the United States and the European Union. Sodium 4-phenylbutyrate is an orphan drug for the treatment of urea cycle disorders. In addition, 4-phenylbutyric acid sodium salt has also been described in patents and in the scientific literature for a number of medical uses. These uses encompass a variety of illnesses , such as benign prostatic hyperplasia, cancer, HIV, kidney failure and thalassemia. For example, WO 95/10271 discloses compositions and methods using 4-phenylbutric acid derivatives for therapy and prevention of a number of pathologies. Furthermore, EP 2 599 767 A1 describes a number of 4-phenylbutric acid derivatives for use in cancer therapy and other pharmaceutical applications. EP 2 389 932 refers to pharmaceutical compositions comprising the histone hyperacetylating agent phenyl-butyric acid for use in the treatment of a genetic disorder, like treatment of depression, vaginitis and varicosis or the prevention of Sudden Infant Death Syndrome with a genetic disorder background. T.A. Gudasheva et al. in a publication in the Russian Journal of bioorganic Chemistry, 2007, Vol. 33, No. 4, pp. 383-389 disclose the design and synthesis of cholecystokinin-4 dipeptide analogues with anxiolytic and anxiogenic activities. However, the scope of medical uses for 4-phenylbutyric acid and derivatives thereof is still not fully explored.

It is a problem underlying the present invention to provide new compounds for use in the treatment of inflammatory diseases, angiopathic diseases, mental disorders and tinnitus.

This problem is solved with 4-phenylbutric acid derivatives according to the independent claims.

The present invention is defined in the claims and refers to particular 4-phenylbutyric acid derivatives of Formula 1, 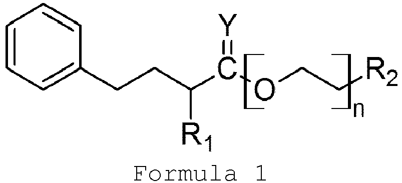 wherein Y is O; R₁ is H; R₂ is a radical of an amino acid; and n is 0 which are defined in more detail below,
for use in the treatment of diseases specified below.

In Formula 1, when Y is selected as O, C=Y corresponds to a carbonyl group (C=O).

Specifically, the claimed 4-phenylbutyric acid derivative can be used in the treatment of inflammatory bowel diseases, in particular in the treatment of Crohn's disease and ulcerative colitis. Furthermore, the 4-phenylbutyric acid derivative can be used in the treatment of inflammatory diseases of the bile ducts, in particular in the treatment of primary sclerosing cholangitis. Moreover, the above 4-phenylbutyric acid derivative can be used in the treatment of inflammatory skin diseases, such as in the treatment of psoriasis. But the 4-phenylbutyric acid derivative can also be used in the treatment of recurrent vulvo-vaginitis.

In another aspect, the present invention refers to said 4-phenylbutyric acid derivative for use in the treatment of angiopathic diseases. In this context, the 4-phenylbutyric acid derivative can be used in the treatment of varicose veins.

Yet another aspect of the present invention refers to said 4-phenylbutyric acid derivative for use in the treatment of depressive disorder, or in the treatment of tinnitus.

In an above-described 4-phenylbutyric acid derivative, R₂ is of the following structure:

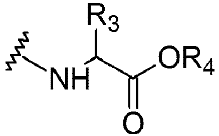

In the above formulas, R₃ is of the following structure: 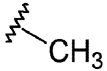 and R₄ is H.

On the other hand, R₂ can be selected from any of the following structures (which are not according to the invention though):

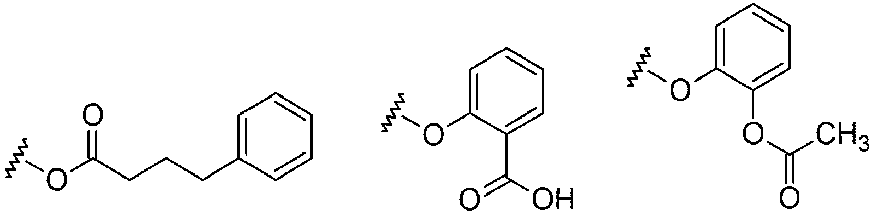

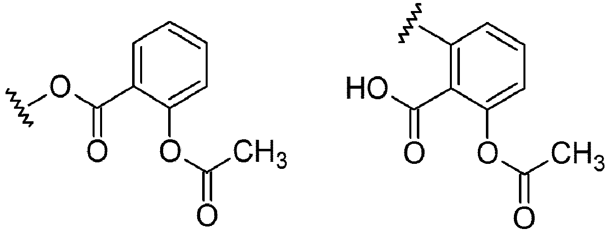

The 4-phenylbutyric acid derivative according to the present invention is selected from any of the following structures: 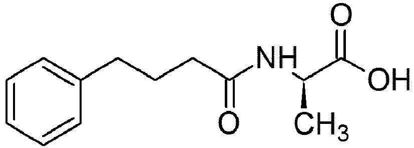 N-(4-phenylbutanoyl)-D-alanine (4-PB)

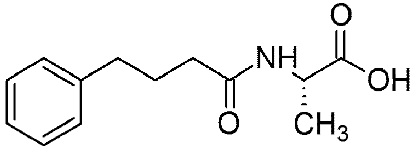

The above-mentioned 4-phenylbutric acid derivatives may be employed as a single stereoisomer or as a mixture of stereoisomers. Such stereoisomers can be enantiomers. The compounds may be used as a racemate. However, preferably they are used in enantiomerically pure form. Furthermore, the 4-phenylbutric acid derivatives may be employed in form of the free acid, the free base, or as a salt.

4-phenylbutric acid derivatives of Formula 1 are physiologically well tolerated.

The present invention also relates to a medicament or a pharmaceutical formulation with a 4-phenylbutric acid derivative according to the invention for use in the treatment of the diseases claimed. The medicament or pharmaceutical formulation can be of any form suitable for the application to humans and/or animals, preferably humans including infants, children and adults. It can be produced by standard procedures known to those skilled in the art. The composition of the medicament or pharmaceutical formulation may vary depending on the route of administration.

The medicament or pharmaceutical formulation of the present invention may for example be administered partially in combination with conventional injectable liquid carriers, such as water or suitable alcohols. Conventional pharmaceutical recipients for injection, such as stabilizing agents, solubilizing agents, and buffers, may be included in such injectable compositions. Such medicaments or pharmaceutical formulations may for example injected intramuscularly, intraperitoneal or intravenously. Medicaments or pharmaceutical formulations according to the present invention may also be formulated into orally administrable compositions containing one or more physiologically compatible carrier or excipient, in solid or liquid form. These compositions may contain conventional ingredients such as binding agents, fillers, lubricants, and acceptable wetting agents. The compositions may take any convenient form, such as tablets, pellets, granule, capsules, lozenges, aqueous or oily solutions, suspensions, emulsions or dry powdered forms suitable for reconstitution with water or other suitable liquid media before use. The multiparticulate forms, such as pellets or granules, may be filled into a capsule, compressed into tablets or suspended in a suitable liquid. Furthermore, suitable controlled release formulations and methods for their preparations are known from the prior art.

Medicaments or pharmaceutical formulations according to the present invention may also comprise enteric coating, so that their dissolution is depended on the pH value. Due to said coating, the medicament may pass the stomach unresolved and the 4-phenylbutric acid derivatives according to the invention are liberated in the intestinal tract. Preferably, the enteric coating is soluble at the pH value of 5.0-7.5. Suitable materials and methods for the preparation are known in the prior art.

Typically, medicaments or pharmaceutical formulations according to the present invention contain 1-60% by weight of one or more 4-phenylbutric acid derivatives according to the invention and 40-99% by weight of one or more auxiliary substances.

4-phenylbutric acid derivatives according to the invention may also be at ministered topically or via a suppository.

The daily dosage for humans and animals may vary depending on factors that have their basis in the respective species or other factors, such as age, sex, weight or degree of illness and so forth. The daily dosage for humans is in the range of 10 mg to 2'000 mg of active substance (4-phenylbutric acid derivativesaccording to the invention), to be administered during one or several intakes per day. Preferably, the daily dosage for humans is in the range of 100 mg to 500 mg of active substance (4-phenylbutric acid derivativesaccording to the invention) to be administered during one or several intakes per day. Even more preferably, the daily dosage for humans is in the range of 300 mg to 400 mg of active substances (4-phenylbutric acid derivatives according to the invention) to be administered in two intakes per day.

Further advantages and aspects of the present invention become apparent from the description of the following examples.

The following examples refer to the *in vivo* biological activity of *N*-(4-phenylbutanoyl)-D-alanine (4-PB). Other embodiments will be evident to a person skilled in the art through the forgoing detailed description.

### Example 1: In vivo activity of 4-PB against ulcerative colitis

A female patient with recurrent episodes of ulcerative colitis showed a good response to 4-PB. After 6 months of treatment, bowels were normal and she had no pain and no diarrhea anymore.

### Example 2: In vivo activity of 4-PB against primary sclerosing cholangitis

Two female patients with primary sclerosing cholangitis were treated with 4-PB. Both had recurrent fever and abdominal pains for days. Both were chosen for liver transplant. After 5 months of treatment with 4-PB both were free of symptoms and signs of the underlying cholangitis.

### Example 3: In vivo activity of 4-PB against psoriasis

A male patient with generalized psoriasis, who has been treated for years with corticoids ointments, received 4-PB for a colon carcinoma. After four months of treatment, the cirrhosis showed a complete remission, lasting after sessions of 4-PB.

### Example 4: In vivo activity of 4-PB against recurrent vulvo-vaginitis

Two female patients were treated with 4-PB for other reasons (one for cancer and one for depressions). After four months of treatment, both were free of vulvo-vaginitis and state in complete remission, even after sessions of 4-PB.

### Example 5: In vivo activity of 4-PB against varicose veins

A female patient with breast cancer was treated with 4-PB. During the therapy for the cancer, a disappearance of the enlarged veins on both legs was realized without a recurrence in the next 10 years. The spider veins on both legs remained unchanged.

### Example 6: In vivo activity of 4-PB against major depressive disorder

Four female patients and one male patient with major depressive disorders, some of which suffering from other diseases, were treated with 4-PB. Surprisingly, depressions disappeared in three to five months. No recurrences have been observed.

### Example 7: In vivo activity of 4-PB against tinnitus

Two male patients with tinnitus, lasting for more than ten years, were treated with 4-PB. After four months of treatment, tinnitus has disappeared in both cases.

## Claims

1. 4-Phenylbutyric acid derivative of Formula 1, 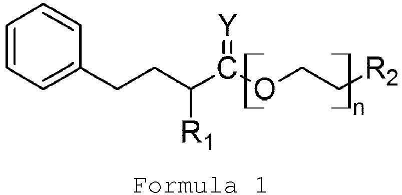 wherein
Y is O;
R₁ is H;
R₂ is a radical of an amino acid; and
n is 0,
and wherein the 4-phenylbutyric acid derivative is selected from any of the following structures 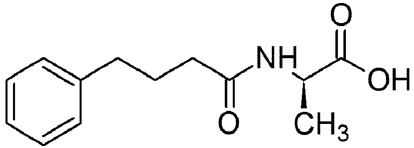 N-(4-phenylbutanoyl)-D-alanine (4-PB); 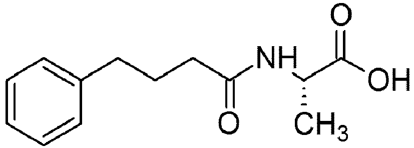 for use in the treatment of inflammatory bowel diseases.

2. 4-Phenylbutyric acid derivative according to claim 1 for use in the treatment of Crohn's disease.

3. 4-Phenylbutyric acid derivative according to claim 1 for use in the treatment of ulcerative colitis.

4. 4-Phenylbutyric acid derivative according to claim 1 for use in the treatment of inflammatory diseases of the bile ducts.

5. 4-Phenylbutyric acid derivative according to claim 1 for use in the treatment of primary sclerosing cholangitis.

6. 4-Phenylbutyric acid derivative according to claim 1 for use in the treatment of psoriasis.

7. 4-Phenylbutyric acid derivative according to claim 1 for use in the treatment of recurrent vulvo-vaginitis.

8. 4-Phenylbutyric acid derivative according to claim 1 for use in the treatment of varicose veins.

9. 4-Phenylbutyric acid derivative according to claim 1 for use in the treatment of major depressive disorder.

10. 4-Phenylbutyric acid derivative according to claim 1 for use in the treatment of tinnitus.

## Patentansprüche

1. 4-Phenylbuttersäurederivat der Formel 1, 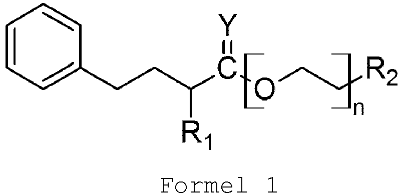 wobei
Y O ist;
R₁ H ist;
R₂ ist ein Rest einer Aminosäure; und
n ist 0,
und wobei das 4-Phenylbuttersäurederivat aus einer der folgenden Strukturen ausgewählt ist 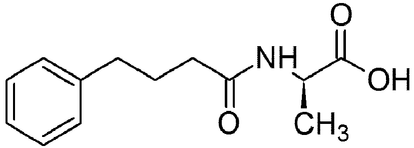 N-(4-Phenylbutanoyl)-D-alanine (4-PB); 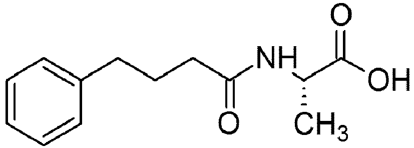 zur Verwendung bei der Behandlung von entzündlichen Darmerkrankungen.

2. 4-Phenylbuttersäurederivat nach Anspruch 1 zur Verwendung bei der Behandlung von Morbus Crohn.

3. 4-Phenylbuttersäurederivat nach Anspruch 1 zur Verwendung bei der Behandlung von Colitis ulcerosa.

4. 4-Phenylbuttersäurederivat nach Anspruch 1 zur Verwendung bei der Behandlung von entzündlichen Erkrankungen der Gallenwege.

5. 4-Phenylbuttersäurederivat nach Anspruch 1 zur Verwendung bei der Behandlung der primär sklerosierenden Cholangitis.

6. 4-Phenylbuttersäurederivat nach Anspruch 1 zur Verwendung bei der Behandlung von Psoriasis.

7. 4-Phenylbuttersäure-Derivat nach Anspruch bei der Behandlung von rezidivierender Vulvo-Vaginitis.

8. 4-Phenylbuttersäurederivat nach Anspruch bei der Behandlung von Krampfadern.

9. 4-Phenylbuttersäurederivat nach Anspruch 1 zur Verwendung bei der Behandlung einer schweren depressiven Störung.

10. 4-Phenylbutyric acid derivative according to claim 1 for use in the treatment of tinnitus.

## Revendications

1. Dérivé de l'acide 4-phénylbutyrique de formule, 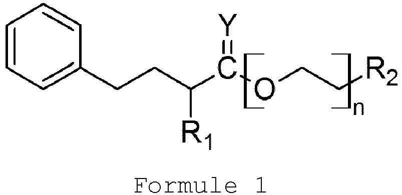 où
Y est O;
R1 est H;
R2 est un radical d'un acide aminé; et
n est 0,
et dans laquelle le dérivé de l'acide 4-phénylbutyrique est choisi parmi l'une quelconque des structures suivantes 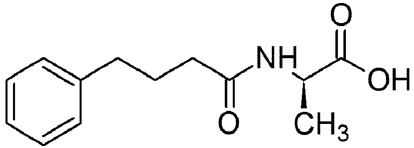 N-(4-phénylbutanoyl)-D-alanine (4-PB); 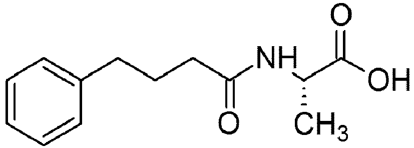 pour une utilisation dans le traitement des maladies inflammatoires de l'intestin.

2. Dérivé de l'acide 4-phénylbutyrique selon la revendication 1 pour une utilisation dans le traitement de la maladie de Crohn.

3. Dérivé de l'acide 4-phénylbutyrique selon la revendication 1 pour une utilisation dans le traitement de la colite ulcéreuse.

4. Dérivé de l'acide 4-phénylbutyrique selon la revendication 1 pour une utilisation dans le traitement des maladies inflammatoires des voies biliaires.

5. Dérivé d'acide 4-phénylbutyrique selon la revendication 1 pour une utilisation dans le traitement de la cholangite sclérosante primaire.

6. Dérivé de l'acide 4-phénylbutyrique selon la revendication 1 pour une utilisation dans le traitement du psoriasis.

7. Dérivé de l'acide 4-phénylbutyrique selon la revendication 1, dans le traitement de la vulvo-vaginite récurrente.

8. Dérivé de l'acide 4-phénylbutyrique selon la revendication 1, dans le traitement des veines variqueuses.

9. Dérivé de l'acide 4-phénylbutyrique selon la revendication 1 pour une utilisation dans le traitement du trouble dépressif majeur.

10. Dérivé de l'acide 4-phénylbutyrique selon la revendication 1 pour une utilisation dans le traitement des acouphènes.
